# EUROPEAN PATENT APPLICATION

(11) **EP 4 278 960 A1**
(43) Date of publication of application: **22.11.2023**
(21) Application number: 22188269.9
(22) Date of filing: 02.08.2022
(51) Int. Cl.: A61B 5/021, A61B 5/024, A61B 5/0245, A61B 5/352, A61B 5/00

(54) **NON-INVASIVE BLOOD PRESSURE MEASUREMENT**

(30) Priority: 20.05.2022 IN 202241029053
(71) Applicant: L & T Technology Services Limited, Chennai 600 089 (IN)
(72) Inventor: SRIKANTH, CHANDRASHEKAR, 570026 Mysore, Karnataka (IN); KEWAL, DESHPANDE, 570023 Mysore, Karnataka (IN); ABHINAV, SHUBHAM, 834001 Hatia, Ranchi, Jharkhand (IN)
(74) Representative: Studio Torta S.p.A.

(57) **Abstract**

A non-invasive method and an apparatus for measuring blood pressure of a subject (101). The method includes capturing and processing a first input (102a) and a second input (103a) to detect a first peak value (Tim (ECG)) and a second peak value (Tim (PPG)). The method includes calculating a pulse transit time (PTT) based on the first peak value and the second peak value, and a pulse wave velocity (PWV) based on the PTT and an arm length of the subject (101). Further, determining whether the PWV is associated with predefined coefficient values stored in the memory (118). In response, initiating a calibration before measuring the blood pressure under varying conditions of the subject (101) to generate a plurality of coefficients (304) based on one or more predefined blood pressure values or determining the blood pressure based on an association of the pulse wave velocity and the predefined coefficient values.

## Description

### TECHNICAL FIELD

The present disclosure relates generally to blood pressure measurement and more particularly to a cuffless non-invasive blood pressure measurement.

### BACKGROUND OF INVENTION

The following description includes information that may be useful in understanding the present disclosure. It is not an admission that any of the information provided herein is prior art or relevant to the presently claimed disclosure, or that any publication specifically or implicitly referenced is prior art.

Conventional sphygmomanometer also known as a blood pressure monitor is an instrument used to measure blood pressure which may work under auscultatory principle. The instrument may include an inflatable cuff to collapse and then release the blood vessels or blood flow under the cuff in a controlled manner, and a mercury manometer may be used to measure and indicate the blood pressure. The sphygmomanometer may be used with a stethoscope to hear Korotkoff sounds. In operation, as the pressure in the cuffs falls, the Korotkoff sounds are heard as the blood flow starts again in the blood vessel under the cuff. The pressure at which this sound is heard is noted and recorded as the systolic blood pressure. The cuff pressure is further released until the sound can no longer be heard. This is recorded as the diastolic blood pressure.

Further, a digital meter may be used to measure the blood pressure, which may work under oscillometry principle that blood flowing through the blood vessel between systolic and diastolic blood pressures causes vibrations which may be detected and transduced into electrical signals. The digital meter uses a cuff which automatically inflates and deflates using an electronic pump. In operation, rising pressure in the cuff eventually stops blood flowing in the blood vessel under the cuff. As the cuff reaches a systolic blood pressure, the blood begins to flow around the blood vessel. This creates a vibration which is detected by a meter, which records the systolic blood pressure. As the cuff continues to deflate, it reaches a diastolic blood pressure, and the vibration stops, which is then detected by the meter recording the diastolic blood pressure.

However, such conventional instruments used to measure the blood pressure may be subjected to errors under certain conditions and environments leading to less accurate blood pressure readings.

### SUMMARY OF INVENTION

The following presents a simplified summary to provide a basic understanding of some aspects of the disclosed material handling system. This summary is not an extensive overview and is intended to neither identify key or critical elements nor delineate the scope of such elements. Its purpose is to present some concepts of the described features in a simplified form as a prelude to the more detailed description that is presented later.

Various example embodiments described herein relate to a non-invasive method for measuring a blood pressure of a subject. The method includes capturing a first input from a first sensor and a second input from a second sensor and processing the first input and the second input to detect a first peak value in the first input and a second peak value in the second input. The first sensor and the second sensor are connected to the subject. The method further includes calculating a pulse transit time (PTT) based on the first peak value and the second peak value, and a pulse wave velocity (PWV) based on the pulse transit time and an arm length of the subject. The pulse transit time is indicative of a time interval of a pulse pressure wave to travel from one blood vessel to another blood vessel of the subject. The pulse wave velocity is indicative of a rate at which the pulse pressure wave travels through the blood vessel. The method further includes determining whether the pulse wave velocity is associated with predefined coefficient values stored in the memory. In response to the determination: initiating a calibration before measuring the blood pressure by repeating steps a) to d) under varying conditions of the subject to generate a plurality of coefficients based on one or more predefined blood pressure values; or determining the blood pressure based on an association of the pulse wave velocity and the predefined coefficient values. Various example embodiments described herein relate to a non-invasive method for measuring a blood pressure of a subject, wherein generating the plurality of coefficients further includes performing a linear regression on the pulse wave velocity and the one or more predefined blood pressure values.

Various example embodiments described herein relate to a non-invasive method for measuring a blood pressure of a subject. The method includes filtering the first input and the second input with a low pass filter and a high pass filter to detect the first peak value and the second peak value. Various example embodiments described herein relate to a non-invasive method for measuring a blood pressure of a subject. The method includes displaying the blood pressure of the subject on a user interface.

Various example embodiments described herein relate to a non-invasive method for measuring a blood pressure of a subject., wherein the blood pressure is indicative of systolic blood pressure or diastolic blood pressure, wherein the systolic blood pressure (SBP) is calculated by SBP = a^{∗}pulse wave velocity + b, wherein the diastolic blood pressure (DBP) is calculated by DBP = c^{∗}pulse wave velocity + d, and wherein a, b, c, and d are the coefficients.

Various example embodiments described herein relate to an apparatus for non-invasively measuring a blood pressure of a subject. The apparatus includes one or more sensors connected to the subject to capture a photoplethysmography waveform and an electrocardiography waveform. A processor communicably coupled to the one or more sensors and a memory, wherein the processor configured to: process the photoplethysmography waveform and the electrocardiography waveform to detect a first peak value in the electrocardiography waveform and a second peak value in the photoplethysmography waveform and calculate a difference in time interval between the first peak value and the second peak value, wherein the difference in time interval is indicative of a time a pulse pressure wave takes to travel in arteries of the subject. The processor further configured to calculate a pulse wave velocity (PWV) based on the difference and an arm length of the subject, wherein the pulse wave velocity is indicative of a rate at which the pulse pressure wave travels through the arteries. The processor further configured to determine whether the pulse wave velocity is associated with predefined coefficient values stored in the memory. When the association exist, the processor further configured to determine a blood pressure of the subject based on the association of the pulse wave velocity and the predefined coefficient values. When the association does not exist, the processor further configured to initiate a calibration by repeating steps a) to c) under varying conditions of the subject to generate a plurality of coefficients based on one or more predefined blood pressure value and determine the blood pressure based on a new pulse wave velocity of the subject and the plurality of coefficients. Various example embodiments described herein relate to an apparatus for non-invasively measuring a blood pressure of a subject. The apparatus includes a visual interface to display the blood pressure, wherein the blood pressure is indicative of systolic blood pressure or diastolic blood pressure, and an audio interface to indicate one of the blood pressure is determined and the calibration is initiated or completed.

Various example embodiments described herein relate to an apparatus for non-invasively measuring a blood pressure of a subject, wherein the processor is further configured to: predict the blood pressure of the subject by executing a machine learning model trained based on multiple pulse flow velocities, one or more predefined blood pressure values, and a plurality of coefficients when the association does not exist.

Various example embodiments described herein relate to an apparatus for non-invasively measuring a blood pressure of a subject. The apparatus includes a low pass filter and a high pass filter to eliminate a noise in the photoplethysmography waveform and the electrocardiography waveform to detect the first peak value and the second peak value.

Various example embodiments described herein relate to an apparatus for non-invasively measuring a blood pressure of a subject, wherein the one or more sensor comprises a first sensor comprising: three leads with a first lead as ground connected to above waist, a second lead as positive connected to left side of subject, a third lead as a negative connected to the right side of the subject, and a second sensor comprising: a light source with a transmitter and a receiver, and wherein the second sensor is connected to one of a finger or a wrist of the subject.

The above summary is provided merely for purposes of summarizing some example embodiments to provide a basic understanding of some aspects of the disclosure. Accordingly, it will be appreciated that the above-described embodiments are merely examples and should not be construed to narrow the scope or spirit of the disclosure in any way. It will be appreciated that the scope of the disclosure encompasses many potential embodiments in addition to those here summarized, some of which will be further described below.

### BRIEF DESCRIPTION OF DRAWINGS

The embodiments of the disclosure itself, as well as a preferred mode of use, further objectives and advantages thereof, will best be understood by reference to the following detailed description of an illustrative embodiment when read in conjunction with the accompanying drawings. One or more embodiments are now described, by way of example only, with reference to the accompanying drawings in which:
FIG. 1 discloses a block diagram of an exemplary apparatus for non-invasive blood pressure measurement, in accordance with an embodiment of the present disclosure.
FIG. 2 discloses a schematic diagram of a first input and the second input obtained from the subject used in non-invasive blood pressure measurement, in accordance with an embodiment of the present disclosure.
FIG. 3 discloses a schematic diagram for calibration by generating a plurality of coefficients used in non-invasive blood pressure measurement, in accordance with an embodiment of the present disclosure.
FIGS. 4A and 4B disclose example look-up tables stored in the memory, in accordance with an embodiment of the present disclosure.
FIGS. 5A and 5B disclose example user interfaces used in non-invasive blood pressure measurement, in accordance with an embodiment of the present disclosure.
FIGS. 6A, 6B and 6C disclose example user interfaces for database entry used in non-invasive blood pressure measurement, in accordance with an embodiment of the present disclosure.
FIG. 7 discloses a flowchart of a method for non-invasive blood pressure measurement, in accordance with an embodiment of the present disclosure.

The figures depict embodiments of the disclosure for purposes of illustration only. One skilled in the art will readily recognize from the following description that alternative embodiments of the structures and methods illustrated herein may be employed without departing from the principles of the disclosure described herein.

### DETAILED DESCRIPTION

Various embodiments of the present invention now will be described more fully hereinafter with reference to the accompanying drawings, in which some, but not all embodiments of the invention are shown. Indeed, the invention may be embodied in many different forms and should not be construed as limited to the embodiments set forth herein. Rather, these embodiments are provided so that this disclosure will satisfy applicable legal requirements. The term "or" is used herein in both the alternative and conjunctive sense, unless otherwise indicated. The terms "illustrative," "example," and "exemplary" are used to be examples with no indication of quality level. Like numbers refer to like elements throughout.

The phrases "in an embodiment," "in one embodiment," "according to one embodiment," and the like generally mean that the particular feature, structure, or characteristic following the phrase may be included in at least one embodiment of the present disclosure and may be included in more than one embodiment of the present disclosure (importantly, such phrases do not necessarily refer to the same embodiment).

The word "exemplary" is used herein to mean "serving as an example, instance, or illustration." Any implementation described herein as "exemplary" is not necessarily to be construed as preferred or advantageous over other implementations.

If the specification states a component or feature "can," "may," "could," "should," "would," "preferably," "possibly," "typically," "optionally," "for example," "often," or "might" (or other such language) be included or have a characteristic, that particular component or feature is not required to be included or to have the characteristic. Such component or feature may be optionally included in some embodiments, or it may be excluded.

Conventional blood pressure instruments such as the sphygmomanometer or the digital meter may be subjected to errors, manual effort and may cause discomfort to subjects on whom these instruments are used. In some examples, the conventional blood pressure instruments require the cuff to be tied tightly around a wrist or an arm of the subject which may cause discomfort to the subject when inflated for a prolonged time leading to numbness and pain in segments of the wrist or the arm around which the cuff is tied.

In some scenarios, when the subject requires regular monitoring of the blood pressure, then the cuff may be tied and untied at each instance the subject is being monitored, which requires manual effort by nurses or doctors or medical staff treating the subject.

In some scenarios, a length of the cuff may also play a crucial role in the blood pressure measurements as the length may change based on a circumference of the arm which may differ for each subject. For example, the subject may be an infant, an adult, or a pet animal with different cuff requirements.

In some scenarios, when using the sphygmomanometer, the Korotkoff sounds heard through the stethoscope appears to be critical in determining the blood pressure, which may be subjected to errors under noisy environments leading to less accurate blood pressure measurements.

In some scenarios, blood pressure may change due to the environment and conditions around the subject. For example, a sensitive subject may experience anxiety or fear when seated in the waiting room of hospital or clinic or while inflating the cuff which may cause errors in the blood pressure readings as the conventional instruments are not calibrated in a manner to handle such scenarios. In some examples, a movement artifact such as a posture and a position of the subject while monitoring the blood pressure may also affect the readings as the conventional instruments are sensitive to such changes which leads to inaccuracy.

Through applied effort, ingenuity, and innovation, many of the above identified problems have been solved by developing solutions that are included in embodiments of the present disclosure, many examples of which are described in detail herein. The present disclosure relates to an apparatus and a method for measuring a blood pressure of a subject using two inputs from one or more sensors. The apparatus includes a processor which is configured to capture a first input and a second input from one or more sensors connected to the subject. The sensor may be a cuffless sensor measuring the first input from the subject as a photoplethysmography (PPG) waveform and second input from the subject as an electrocardiography (ECG) waveform. The processor is further configured to a pulse transit time (PTT), a pulse wave velocity (PWV), and a plurality of coefficients based on the pulse wave velocity. The pulse transit time is the difference in time interval between the first peak value and the second peak value obtained from the waveforms and the pulse wave velocity is calculated based on the difference in the time interval and an arm length of the subject. The processor is further configured to determine the blood pressure of the subject based on the pulse wave velocity and the plurality of coefficients. In this regard, the apparatus may use the measurements from the ECG and PPG waveform to detect the blood pressure obviating the need to use any cuff based conventional instruments.

According to an embodiment, the apparatus is calibrated under varying conditions of the subject in which the processor may execute the steps related to calculating the pulse wave velocities for several iterations under varying conditions of the subject to generate the plurality of coefficients for the subject. In this regard, the apparatus may be calibrated to determine the blood pressure obviating inaccuracies imposed when there is a movement artifact.

According to an embodiment, the apparatus detects the blood pressure using the ECG and PPG waveform to detect the blood pressure. The processor is configured to determine whether the pulse wave velocity is associated with predefined coefficient values stored in the memory, wherein when the association exist, determine a blood pressure of the subject based on the pulse wave velocity and the predefined coefficient values, and wherein when the association does not exist, initiate a calibration process to generate the plurality of coefficients based on the pulse wave velocity and predefined blood pressure values under varying conditions of the subject. In this regard, the apparatus may be used for making a decision either to measure the blood pressure or to calibrate and measure the blood pressure for the subject under evaluation. Therefore, the apparatus may provide error free readings even with change in the environment around the subject and conditions of the subject.

In the following detailed description of exemplary embodiments of the disclosure, specific representative embodiments in which the disclosure may be practiced are described in sufficient detail to enable those skilled in the art to practice the disclosed embodiments. For example, specific details such as specific method orders, structures, elements, and connections have been presented herein. However, it is to be understood that the specific details presented need not be utilized to practice embodiments of the present disclosure. It is also to be understood that other embodiments may be utilized and that logical, architectural, programmatic, mechanical electrical and other changes may be made without departing from the general scope of the disclosure. The following detailed description is, therefore, not to be taken in a limiting sense, and the scope of the present disclosure is defined by the appended claims and equivalents thereof.

As used herein, the term "apparatus" encompasses all apparatus, devices, and machines for processing data, including by way of example a programmable processor, a computer, or multiple processors or computers. The apparatus can include, in addition to hardware, code that creates an execution environment for the computer program in question, e.g., code that constitutes processor firmware, a protocol stack, a database management system, an operating system, a cross-platform runtime environment, or a combination of one or more of them. In addition, the apparatus can employ different computing model infrastructures, such as web services, distributed computing, and grid computing infrastructures.

As used herein, the terms such as "store," "storage," "data store," data storage," "database," and substantially any other information storage component relevant to operation and functionality of a component are utilized to refer to "memory components," entities embodied in a "memory," or components comprising a memory. It is to be appreciated that memory and/or memory components described herein can be either volatile memory or nonvolatile memory or can include both volatile and nonvolatile memory.

As used herein, the term "unit" may indicate a controller, microprocessor, and/or custom logic circuit adapted to execute programming code, software, and/or functions implemented in the form of a code that is executable. As used herein, the term "controller" refers to any type of processor, such as a central processing unit (CPU) or processor, microprocessor, or embedded microcontroller. In various embodiments, a "unit" may be implemented mechanically or electronically. For example, the unit may comprise dedicated circuitry or logic that is permanently configured (e.g., as a special-purpose processor, such as a field programmable gate array (FPGA) or an application-specific integrated circuit (ASIC)) to perform certain operations. The unit may also comprise programmable logic or circuitry (e.g., as encompassed within a general-purpose processor or other programmable processor) that is temporarily configured by software to perform certain operations. It will be appreciated that the decision to implement the unit mechanically, in dedicated and permanently configured circuitry, or in temporarily configured circuitry. Accordingly, the term "unit" should be understood to encompass a tangible entity, be that an entity that is physically constructed, permanently configured (e.g., hardwired), or temporarily configured (e.g., programmed) to operate in a certain manner or to perform certain operations described herein. As used herein, "unit" may refer to a hardware module.

As used herein, the term 'machine learning model' or 'deep learning model' or 'ML model' or 'DL model' may be used interchangeably, and these terms shall not be taken in a sense to limit the scope of the present disclosure. Further, the term 'machine learning model' or 'deep learning model' may refer to a model which is trained or is subject to training by the machine learning processor. Further the term 'trained machine learning model' may refer to a model which is trained and ready to be used in the blood pressure measurement, and these terms shall not be taken in a sense to limit the scope of the present disclosure. In some examples, the machine learning model may be supervised, semi-supervised, unsupervised and reinforcement type machine learning model.

As used herein, the terms "first input" and "second input" refers to signals from the first sensor (i.e., ECG sensor) and the second sensor (i.e., PPG sensor). Throughout the description, the terms "first input" and "second input" may be collectively referred to as "signals" or "waveforms" which are produced from the first sensor and the second sensor, and the collective terms "signals" and "waveforms" shall not be taken in a sense to limit the scope of the present disclosure. Throughout the description, the term "first input" and the term "ECG waveform"; and the term "second input" and the term "PPG waveform" may be used interchangeably, and these terms shall not be taken in a sense to limit the scope of the present disclosure.

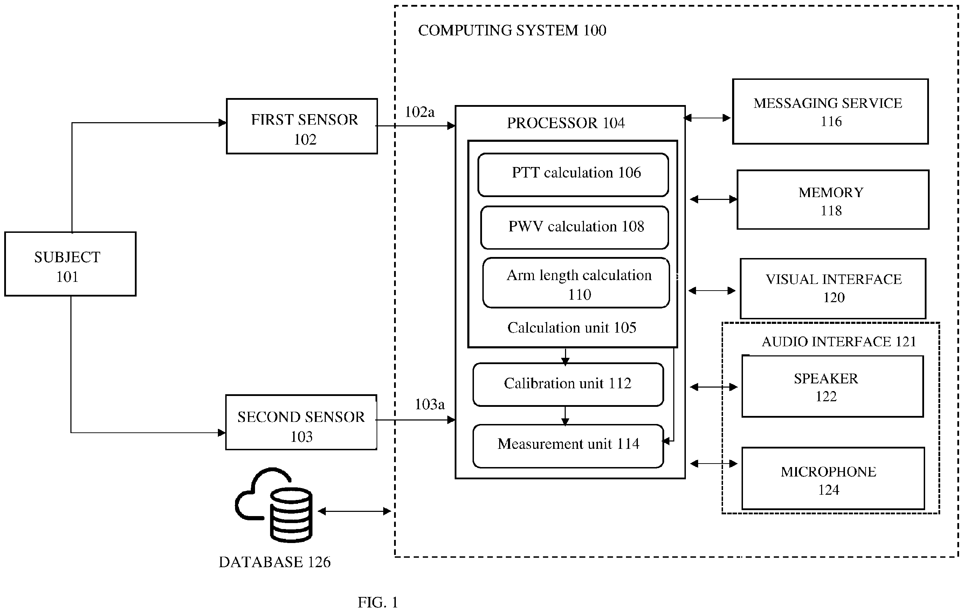

FIG. 1 illustrates a block diagram of an exemplary apparatus for non-invasive blood pressure measurement, in accordance with an embodiment of the present disclosure. The apparatus as shown in FIG. 1 is an example of a cuffless apparatus in which a requirement of using a cuff to measure blood pressure of a subject 101 is obviated. Such an apparatus may be a standalone apparatus or implemented as a part of any wearable or non-wearable electronic device or another medical device. For example, the apparatus may be a part of the wearable device such as a wristwatch or a fitness band. For example, the apparatus may be a part of the non-wearable device such as a mobile phone. For example, the apparatus may be a part of another medical device such as an electroencephalogram (EEG) machine, CT, MRI, or PET scan machine. For example, the apparatus may be a standalone apparatus such as a digital blood pressure monitor without use of the cuff.

According to an embodiment, the apparatus includes one or more sensors 102, 103 and a computing system 100. According to another embodiment, the one or more sensors 102, 103 may be integrated with the computing system 100. In some examples, the computing system 100 may be a microcontroller unit (MCU). According to an embodiment, the apparatus may include the microcontroller unit (MCU) and the computing system 100 which are communicably coupled to each other using a suitable communication channel for processing data from the one or more sensors 102, 103. In some examples, the MCU may be configured to execute a set of instruction and the computing system 100 may be configured to execute another set of instructions to determine the blood pressure of the subject 101. As used herein, a "set of instructions" may refer to programming code, software, and/or functions implemented in the form of a code that is executable by a microcontroller, microprocessor, and/or custom logic circuit adapted to execute these instructions. In some embodiments, these instructions may comprise device drivers, control software, and/or machine code.

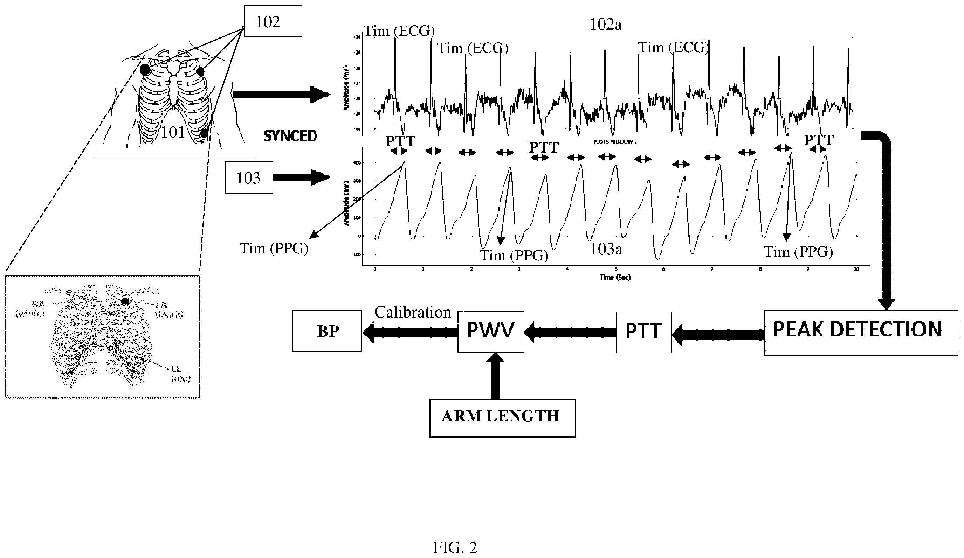

According to an embodiment, the one or more sensors 102, 103 may be connected to a subject 101 for evaluating the blood pressure. The one or more sensors 102, 103 include a first sensor 102 and a second sensor 103. According to an embodiment, the first sensor 102 provides a first input 102a and the second sensor 103 provides a second input 103a to the computing system 100. The first input 102a may be an electrocardiography (ECG) waveform and the second input 103a may be a photoplethysmography (PPG) waveform. In some examples, the first sensor 102 may be an ECG sensor and the second sensor 103 may be a PPG sensor. As shown in the example of FIG. 2, the ECG sensor may include at least three leads LL, LA, RA connected to the subject 101. Further, the second sensor 103 may include a light source with a transmitter and a receiver. The second sensor 103 may be connected to one of a finger or a wrist of the subject 101. According to an embodiment, the first sensor 102 and the second sensor 103 may be integrated in the computing system 100. For example, an embedded chip in the computing system 100 may include the first sensor 102 and the second sensor 103. For example, the MCU and the sensors 102, 103 may be in the form of single integrated circuit. In this regard, the apparatus may be implemented in any wearable electronic device to measure the blood pressure.

According to an embodiment, the computing system 100 may be communicably coupled to the first sensor 102 and the second sensor 103 using suitable communication channels and/or Input/Output (I/O) interfaces. In some examples, the I/O interfaces may include a variety of software and hardware interfaces, for example, a USB, Wi-Fi port, and the like. The I/O interfaces may enable computing system 100 to communicate with other computing systems 100, such as web servers and external databases 126. The I/O interface may facilitate multiple communications within a wide variety of networks and protocol types, and wireless networks, such as WLAN, cellular, or satellite. The I/O interface may include one or more ports for connecting the sensors to the computing system 100 and other external devices. In some examples, the communication channels may be wireless such as Bluetooth or Wi-Fi or wired.

According to an embodiment, the computing system 100 may include a processor 104, a memory 118, a visual interface 120, an audio interface 121, and a messaging service 116. In some examples, the processor 104 may be implemented as one or more microprocessor, microcomputers, microcontrollers, digital signal processor 104s, central processing units, state machines, logic circuitries, and/or any devices that manipulate signals based on operational instructions. Among other capabilities, the processor 104 may be configured to fetch and execute computer-readable instructions stored in the memory 118. In some examples, the processor 104 may also operate to support performance of relevant operations in a cloud computing environment or as a software as a service (SaaS). For example, at least some of the operations may be performed in the cloud computing environment, these operations being accessible via a network (e.g., the Internet) and via one or more appropriate interfaces (e.g., application program interfaces (APIs).

By way of example but not limitation, the memory 118 may include random access memory (RAM), read-only memory (ROM), electrically erasable programmable read-only memory (EEPROM), FLASH memory, disk storage, magnetic storage devices, or the like. Disk storage, as used herein, includes compact disc (CD), laser disc, optical disc, digital versatile disc (DVD), floppy disk, and Blu-ray Disc^{™}, or other storage devices that store data magnetically or optically with lasers. Combinations of the above types of media are also included within the scope of the terms non-transitory computer-readable and processor readable media. Additionally, any combination of instructions stored on the one or more non-transitory processor readable or computer-readable media may be referred to herein as a computer program product.

According to an embodiment, the processor 104 may include a calculation unit 105, a calibration unit 112, and a measurement unit 114. The calculation unit 105 may be configured to perform one of a Pulse Transit Time (PTT) calculation 106, Pulse Wave Velocity (PWV) calculation 108 and Arm Length calculation 110 or their combination. In some examples, these units may also operate in a cloud computing environment to support calculations for the blood pressure measurement.

In some examples, the visual interface 120 and the audio interface 121 are user interfaces that may allow human users to communicate commands to an application installed on the computing system 100 and receive outputs from the application. By way of example but not limitation, the audio interface 121 may accept speech-based inputs and present audio outputs. By way of example but not limitation, the audio interface 121 may be an alarm system that may be used to audibly monitor a condition of the subject 101 during an alarm condition such as a sudden rise or fall in the blood pressure measurement. In some examples, the alarm system may be used to dispatch a verbal request for help to a surrounding environment of the subject 101 when the alarm condition exist. In this regard, the audio interface 121 may include a speaker 122 to output the alarm and the audio outputs. In some examples, the audio interface 121 may accept speech-based inputs via a microphone 124. In some examples, speech-based inputs may trigger the blood pressure measurement. For example, the speech-based input such as "What is my BP?" may be processed by a speech recognition engine or a speech recognition application software which converts acoustical signals resulting from spoken words to digital signals recognizable by a dedicated software application running in the computing system 100 to trigger the blood pressure measurement.

In some examples, the visual interface 120 may be a graphically generated user interface on a display, such as a light emitting diode (LED) display, liquid crystal display (LCD), a cathode ray tube (CRT), or any other display suitable for conveying information related to the blood pressure measurement. The display may have a touch-sensitive interface with graphics including icons, text, web sites, animations, videos, electronic images, and any other forms of graphics providing interactive information to the human users. In some examples, the visual interface 120 may include one or more graphical user interfaces which may be activated and selectively displayed by means of a menu selection, a virtual button, a physical button or at least one predetermined movement on the touch-sensitive interface.

In some examples, the messaging service 116 may include Short Message Service (SMS) or Multimedia Messaging service (MMS) capable of communicating messages over a cellular network. In some examples, the messaging service 116 may include messaging applications such as but not limited to Yahoo Messenger, Skype, MS Teams, FaceTime, WhatsApp, and the like. In this regard, the messaging service 116 may be used to send alert messages (e.g., SMS or WhatsApp messages) to one or more users related to the subject 101 based on the alert condition. The alert message may be displayed on the user's mobile device or computing system 100 through any cloud push mechanisms such that the alert message is not missed or ignored by the users.

According to an embodiment, the computing system 100 is communicably coupled to an external database 126 using suitable communication channels and/or Input/Output (I/O) interfaces. Although the database 126 is shown as an external device, the database 126 may be internal to computer system mounted on a hard disk therein. In some examples, the databases 126 may be implemented by any quantity of any type of conventional or other databases (e.g., network, hierarchical, relational) or storage structures (e.g., files, data structures, web-based storage, disk, or other storage) and the like. The databases 126 may store any desired information arranged in any fashion (e.g., tables, hierarchical, relations, lookups), and may store additional information such as metadata. In some examples, the database 126 can be part of a cloud computing environment.

In operation, as shown in the example embodiment of FIG. 1, the first sensor 102 and the second sensor 103 may sense the first input 102a and the second input 103a from the subject 101 under evaluation. As previously discussed, the first input 102a may be the ECG waveform and the second input 103a may be the PPG waveform. According to an embodiment, the processor 104 captures the first input 102a and the second input 103a in the form of electrical signals from the first sensor 102 and the second sensor 103 and displays it in the form of the ECG waveform and the PPG waveform. For example, electrical currents are produced from the heart of the subject 101 throughout the body during pumping of the blood. The electrical currents may create different potentials at various points in the body, which can be sensed by the first sensor 102 (e.g., electrodes) placed on the skin of the subject 101. The electric potential changes occurring between electrodes placed on the subject 101 demonstrate cardiac activity of the subject 101 which is captured and displayed as the ECG waveform. On the other hand, the second sensor 103 (e.g., a pulse oximeter with a light source and a photodetector) attached to the subject 101 uses a light-based technology which measures light transmission or reflection to sense the rate of blood flow during pumping of the blood. The variations in the attenuation that light undergoes on its path is captured and displayed as the PPG waveform.

According to an embodiment, as shown in FIG. 2, the first sensor 102 includes three leads with a first lead (LL) as ground connected to above waist of the subject 101, a second lead (LA) as positive connected to left side of subject 101, a third lead (RA) as a negative connected to the right side of the subject 101. Specifically, the RA electrode lead is placed under right clavicle, mid-clavicular line within the rib cage frame, the LA electrode lead is placed under left clavicle, mid-clavicular line within the rib cage frame and the LL electrode lead is placed on the lower left abdomen within the rib cage frame. The second sensor 103 includes a light source with a transmitter and a receiver, wherein the second sensor 103 is connected to one of a finger or a wrist of the subject 101. As discussed previously, the first input 102a is displayed as the ECG waveform. The ECG waveform, for example, is a combination of the Q wave, R wave and S wave, commonly known as the "QRS complex". As generally known in the art, this complex waveform may correspond to depolarization of the right and left ventricles of the heart and contraction of large ventricular muscles. The duration or time interval of the QRS complex may decide the conditions of the subject 101. For example, a QRS duration of greater than a predefined seconds (e.g., 0.12 seconds) may be considered as abnormal. As discussed previously, the second input 103a is displayed as the PPG waveform. The PPG waveform, for example, is a combination of peaks and valleys. As generally known in the art, heart rate variability (HRV) may be calculated by time intervals between consecutive peaks in the PPG waveform.

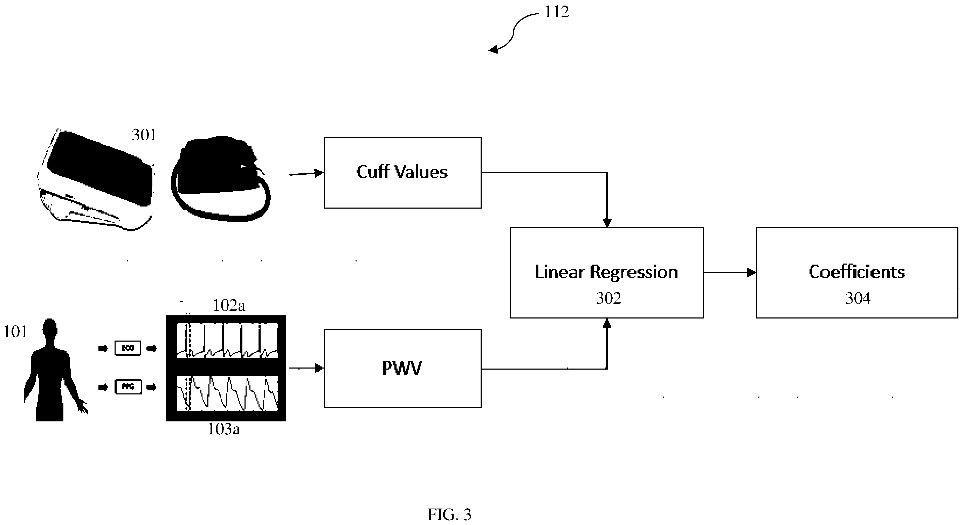

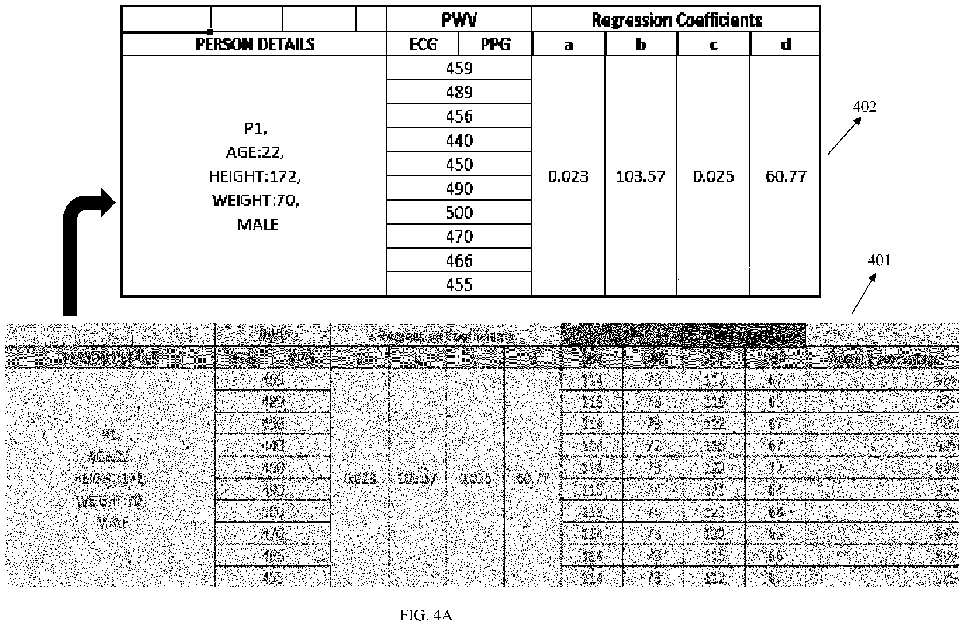

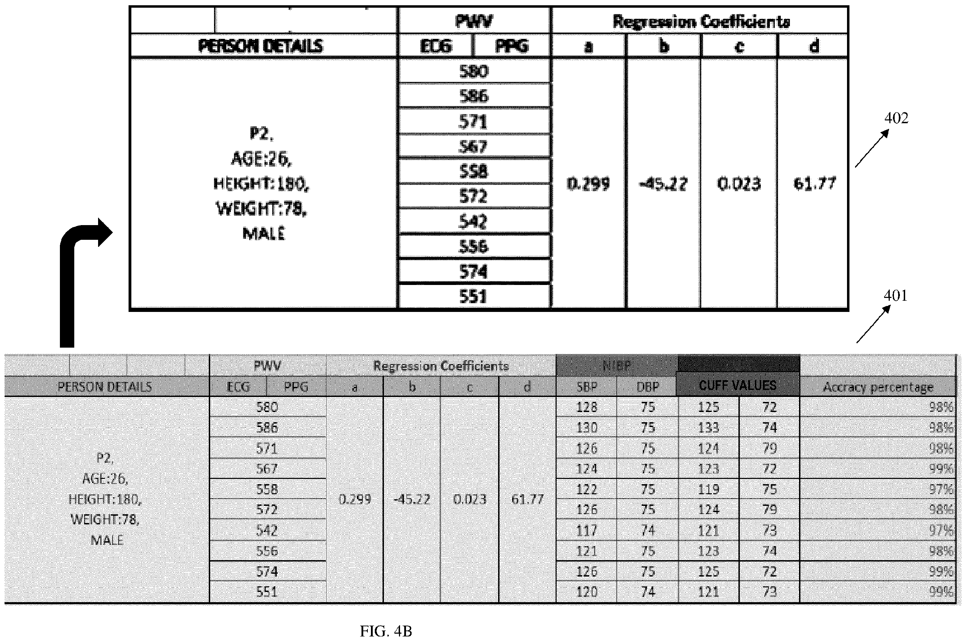

According to an embodiment, the processor 104 may capture the ECG signals and the PPG signals from the first sensor 102 and the second sensor 103 to determine the pulse transit time (PTT), the pulse wave velocity (PWV), and the blood pressure (BP) of the subject 101. As discussed previously, the processor 104 may include the calculation unit 105, the calibration unit 112, and the measurement unit 114 to determine the pulse transit time (PTT), the pulse wave velocity (PWV), and the blood pressure (BP) of the subject 101. The techniques for determining the PTT value and the PWV value using the calculation unit 105 to determine the BP will be disclosed in conjunction with FIG. 2. Further, the techniques for calibration by generating a plurality of coefficients 304 using the calibration unit 112 to determine the blood pressure will be disclosed in conjunction with FIG. 3. Furthermore, the techniques for determining the blood pressure of the subject 101 using the measurement unit 114 will be disclosed in conjunction with FIGS. 4 to 5B.

Turning now to FIG. 2, the signals from the first sensor 102 and the second sensor 103 are displayed as the ECG waveform and the PPG waveform respectively. The X- coordinate may represent an amplitude of the waveform and the Y-coordinate may represent the duration or time interval of the waveform. In some examples, the two waveforms (i.e., the ECG waveform and the PPG waveform) are synchronized using a reference frequency signal either automatically or manually. Such synchronization may be required to match the frequency of the two waveforms to detect a first peak value (Tim (ECG)) and a second peak value (Tim (PPG)). The first peak value (Tim (ECG)), for example, may be a peak point of the R wave of the QRS complex. The second peak value (Tim (PPG)), for example, may be a systolic peak point of the PPG waveform. In some examples, the synchronization may be performed on peak points for the ECG waveform and the PPG waveform to sequence by occurrence time. According to an embodiment, the computing system 100 may include a filter to remove noise from the first input 102a and the second input 103a to detect the first peak value (Tim (ECG)) and the second peak value (Tim (PPG)). The filter, for example, may be a band pass filter or a combination of high pass filter and low pass filter. In some examples, the high pass filter may be used for removing a baseline wander which is a low-frequency noise existing in signals (i.e., ECG or PPG) from the first sensor 102 and the second sensor 103. In some examples, the low pass filter may be used for removing the high-frequency noise existing in signals (i.e., ECG or PPG). Such synchronization and filtering may be required to accurately determine the peak values on the waveforms and ensure that the signals are generated from a same heartbeat cycle or cardiac cycle.

According to an embodiment, the processor 104 may detect the first peak value (Tim (ECG)) in the first input 102a and the second peak value (Tim (PPG)) in the second input 103a. In some examples, the processor 104 may detect the peak values after synchronization and filtering of the first input 102a and the second input 103a. According to an embodiment, the processor 104 may calculate a pulse transit time (PTT) based on the first peak value (Tim (ECG)) and the second peak value (Tim (PPG)). The first peak value may be from the ECG waveform and the second peak value may be from the PPG waveform. For example, the calculation unit 105 may execute the PTT calculation in response to detecting the first peak value (Tim (ECG)) of the ECG waveform and the second peak value (Tim (PPG)) of the PPG waveform. The calculation unit 105 may execute a calculation program when it receives the first input 102a and the second input 103a to calculate a difference in time interval between the first peak value (Tim (ECG)) and the second peak value (Tim (PPG)). For example, the calculation program may include a logic predefined as PTT = diff = Tim (PPG) - Tim (ECG), wherein Tim (ECG) may be the first peak value and the Tim (PPG) may be the second peak value. In other words, the PTT is the time interval between adjacent peak points of the ECG waveform and PPG waveform in the same cardiac cycle. In some examples, the PTT is indicative of a time interval of a pulse pressure wave to travel from one blood vessel to another blood vessel of the subject 101. The blood vessel, for example, may be arteries of the subject 101. In this regard, the processor 104 may detect the peak points in both the waveform and calculate the difference between the R peak of the ECG waveform and systolic peak of the PPG waveform to calculate the PTT value.

According to an embodiment, the processor 104 may calculate a pulse wave velocity (PWV) based on the pulse transit time and an arm length of the subject 101. For example, the calculation unit 105 may execute the PWV calculation in response to detecting the PTT value. The calculation unit 105 may execute a calculation program when it determines the PTT value. For example, the calculation program may include a logic predefined as PWV = Arm Length / PTT, wherein the arm length of the subject 101 is measured using the formula Arm length = 0.55^{∗}height predefined in the calculation program. According to an embodiment, the calculation unit 105 may be communicably coupled to the audio interface 121 and the visual interface 120 to receive real-time inputs which can be used in execution of the calculation program. For example, the calculation unit 105 may transmit a voice prompt to the audio interface 121 requesting the subject 101 or attendant of the subject 101 to input the height of the subject 101 through verbal utterances. The utterances may be processed by a speech recognition engine or a speech recognition application software which converts acoustical signals resulting from spoken words to digital signals recognizable by the calculation unit 105 to trigger the arm length measurement. In some examples, the calculation unit 105 may transmit a pop-up screen/window to the visual interface 120 requesting the subject 101 or the attendant of the subject 101 to input the height of the subject 101 through the touch screen display to trigger the arm length measurement. According to an embodiment, the calculation unit 105 may execute the PWV calculation in response to detecting the PTT value and calculating the arm length. In some examples, the pulse wave velocity is indicative of a rate at which the pulse pressure wave travels through the blood vessels. The blood vessels, for example, may be the arteries of the subject 101. In this regard, the processor 104 may calculate the PTT value and calculate the arm length of the subject 101 to calculate the PWV value.

According to an embodiment, the processor 104 may measure the blood pressure of the subject 101 based on an output from the calibration unit 112. For example, the calibration unit 112 may execute a calibration program including a linear regression model 302 to determine a plurality of coefficients 304 used for measuring the blood pressure of the subject 101. In some examples, the plurality of coefficients 304 may vary from one subject to another subject. In some examples, the plurality of coefficients 304 may vary based on conditions of the subject 101. Turning now to FIG. 3, a schematic diagram for calibration by generating the plurality of coefficients 304 for measuring the blood pressure is shown. In some examples, the plurality of coefficients 304 are regression coefficients which is an outcome of executing the linear regression model 302. For example, the linear regression model 302 may be a model with a single explanatory variable. It may include two-dimensional sample points with one independent variable and one dependent variable (conventionally, the x and y coordinates in a Cartesian coordinate system) and finds a linear function (a non-vertical straight line) that predicts the dependent variable values as a function of the independent variable. In some examples, input parameters for the linear regression model 302 may be the PWV value from the calculation unit 105 and one or more predefined blood pressure values stored in the memory 118. In some examples, the one or more predefined blood pressure values may be pre-calculated using a cuff-based blood pressure equipment 301 and stored in the memory 118. According to an embodiment, the calibration unit 112 may perform linear regression 302 on the PWV value and the one or more predefined blood pressure values to generate the plurality of coefficients 304. In this regard, the processor 104 may generate the plurality of coefficients 304 based on one or more predefined blood pressure values and the PWV value.

According to an embodiment, the processor 104 may perform the calibration under varying conditions of the subject 101. For example, the conditions of the subject 101 may indicate a different physical activity performed by the subject 101 during the calibration. The physical activity may include, for example, sitting, jogging, walking on a treadmill, riding a bicycle and so on. During physical activity, the blood pressure of the subject 101 may increase from about 120/80 mmHg to about 160-220 mmHg. In this regard, the first input 102a and the second input 103a from the first sensor 102 and the second sensor 103 may vary based on the physical activity performed by the subject 101. This may result in change in the PTT value and the PWV value. Therefore, the calibration may include recording plurality of PTT values and PWV values under varying conditions of the subject 101. According to an embodiment, the calibration unit 112 calculates an average PWV value in response to receiving multiple PWV values from the subject 101 under varying conditions. For example, the calibration unit 112 may execute a logic predefined as average PWV=sum(PWV)/len(PWV), wherein the sum(PWV) is a summation of PWV values under varying conditions. The calibration unit 112 may execute the linear regression model 302 using the average PWV and the one or more predefined blood pressure values to generate the plurality of coefficients 304. For example, about 20 values of PWV may be required for the calibration. An average of the 20 values may be determined using the calibration logic. Further, the cuff-based equipment can be used to determine the one or more predefined blood pressure values, which can be used in the calibration logic for the calculation of the regression coefficients 304. The calibration unit 112 may perform the linear regression 302 on the cuff-based blood pressure values and the average PWV to generate the regression coefficients 304.

According to an embodiment, the calibration unit 112 may be communicably coupled to the audio interface 121 and the visual interface 120 to receive real-time inputs which can be used in execution of the calibration program. For example, the calibration unit 112 may transmit a voice prompt to the audio interface 121 requesting the subject 101 or the attendant of the subject 101 to input the cuff-based blood pressure values of the subject 101 through verbal utterances. The utterances may be processed by a speech recognition engine or a speech recognition application software which converts acoustical signals resulting from spoken words to digital signals recognizable by the calibration unit 112 to trigger the execution of the linear regression model 302. In some examples, the calibration unit 112 may transmit a pop-up screen/window to the visual interface 120 requesting the subject 101 or the attendant of the subject 101 to input the cuff-based blood pressure values through the touch screen display to trigger the execution of the linear regression model 302. In some examples, the cuff-based blood pressure values may be predefined in the memory 118. In some examples, the cuff-based blood pressure values may be measured when different physical activity is performed by the subject 101. In some examples, the audio interface 121 and the visual interface 120 may prompt the subject 101 to perform the different physical activity at regular time intervals in a sequence to measure the blood pressure when performing the physical activity.

According to an embodiment, the processor 104 may measure the blood pressure of the subject 101 based on an output from the calibration unit 112. The output may be regression coefficients 304 and the PWV value. For example, the measurement unit 114 may measure the blood pressure of the subject 101 in response to receiving the regression coefficients 304 and the average PWV value. The measurement unit 114 may execute a measurement program when it receives the regression coefficients 304 and the average PWV value. For example, the measurement program may include a logic predefined as BP = a^{∗}PWV + b or BP = c^{∗}PWV + d, wherein a, b, c, d are the regression coefficients 304 generated as the output of executing the linear regression model 302. In some examples, the regression coefficients a and b are used for measuring the systolic blood pressure of the subject 101 and the regression coefficients c and d are used for measuring the diastolic blood pressure of the subject 101.

According to an embodiment, the PWV value may be the average PWV value or a new PWV value different from the average PWV value of the subject 101. In some examples, the new PWV value may be calculated by executing the calculation program using a new PTT value and the arm length. In such a scenario, the calculating unit may communicate the new PWV value to the measurement unit 114 to measure the blood pressure based on the new PWV value and the regression coefficients 304 generated by the calibration unit 112. In this regard, the measurement unit 114 may execute the logic predefined as BP = a^{∗}PWV + b or BP = c^{∗}PWV + d, wherein a, b, c, d are the regression coefficients from the calibration unit 112 and the PWV is the new PWV from the calculation unit 105. Therefore, the processor 104 may measure the blood pressure of the subject 101 based on an output from the calibration unit 112 and the calculation unit 105.

Turning now to FIGS. 4A and 4B which disclose example lookup tables 401, 402 stored in the memory 118 during the calibration, in accordance with an embodiment of the present disclosure. For example, the calibration unit 112 may store the plurality of coefficients 304 in the memory 118 in the form of a look up table 401 after performing the linear regression along with the PWV values. The PWV values may be average PWV values under varying conditions of the subject 101. As shown in FIGS 4A and 4B, the look up table 401 includes multiple fields such as person details of the subject 101, PWV values of the subject 101, regression coefficients 304 of the subject 101, cuff-based predefined blood pressure values, current blood pressure values after calibration, accuracy percentage of the current blood pressure values when compared to the cuff-based predefined blood pressure values.

In some examples, the look up table may be analyzed as follows: the processor 104 performs the linear regression on the PWV values of the subject 101 in the second column of the lookup table 401 and the cuff-based predefined blood pressure values in the ninth and tenth column of the lookup table 401 to generate the regression coefficients populated in columns three to six. Further, the processor 104 may measure the systolic and diastolic blood pressure of the subject 101 based on the PWV values and the regression coefficients, which is populated in columns seven and eight. Furthermore, the processor 104 may optimally perform a comparison between columns seven/nine and eight/ten to determine the percentage accuracy of the calibration, which is populated in the eleventh column.

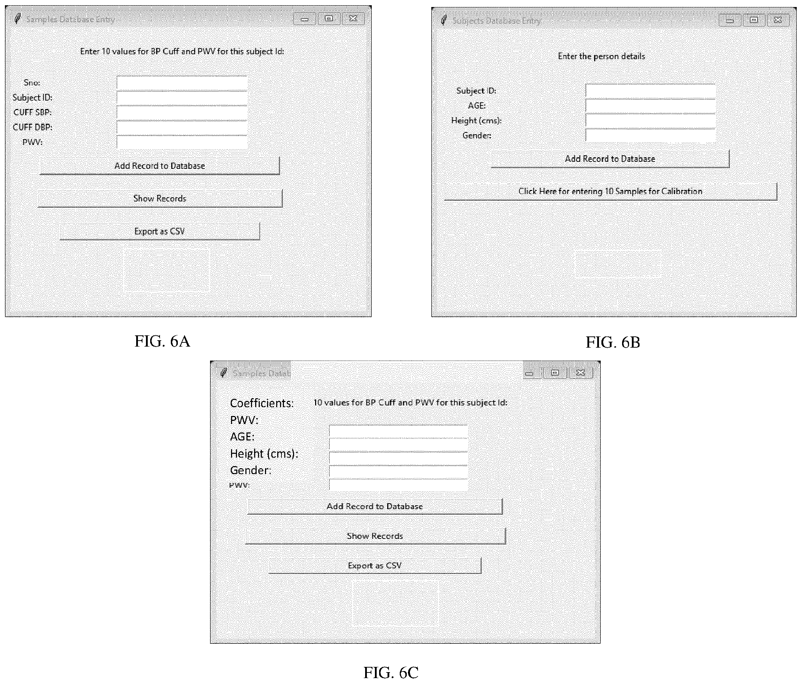

In some examples, as shown in FIG. 4A and FIG. 4B, a portion of the records from the lookup table 401 may be divided and stored in the memory 118 or the external database 126 as a second lookup table 402. For example, the records for fields such as the person details of the subject 101, the PWV values of the subject 101, and the regression coefficients 304 of the subject 101 may be stored in the external database 126 as the second lookup table 402. In some examples, the records in the lookup tables may be manually entered via the visual interface 120 which provides a user interface screen to enter required details for each field in the lookup table. FIGS. 6A to 6C shows examples of the user interface screens to enter the records manually to populate the lookup tables stored in the external database 126. For example, FIG. 6A provides a screen displaying the fields such as subject ID, cuff SBP, cuff DBP, and PWV; FIG. 6B provides a screen displaying the fields such as subject ID, age, height, and gender; and FIG. 6C provides a screen displaying the fields such as regression coefficients, PWV, age, height, and gender. In some examples, each of the screens shown in FIGS. 6A to 6C may be used for creating records either on a single lookup table or individual lookup tables. For example, the second lookup table in FIGS. 4A and FIG. 4B is populated using the user interface screen shown in FIG. 6C which displays the fields such as the regression coefficients, PWV, age, height, and gender. Records in different lookup tables may be logically linked by a lookup field. A lookup field is a primary key common to two or more lookup tables. For example, the lookup field common to the lookup tables of FIGS. 4A and 4B may be the PWV value. In some examples, lookup field may be the person details which includes height, weight, age, and gender. In this regard, the processor 104 may call a lookup field to retrieve meaningful data from multiple lookup tables required to execute the calculation program, calibration program or the measurement program. In this regard, a single lookup table or multiple lookup tables are created and utilized by the processor 104 during and after the calibration.

According to an embodiment, the processor 104 may measure the blood pressure of the subject 101 based on the PWV value and predefined coefficient values. As discussed previously, the predefined coefficient values may be stored in the external database 126 or in the memory 118 after the calibration. For example, when the calculation unit 105 calculates the PWV value of the subject 101, a decision is formulated by the processor 104 either to perform the calibration or to perform the blood pressure measurement without the calibration. In other words, the processor 104 may automatically decide to bypass the calibration unit 112 based on the PWV value outputted from the calculation unit 105. In such a scenario, the processor 104 may instruct the calculation unit 105 to transmit the PWV value to the measurement unit 114 to determine the blood pressure using the PWV value.

According to an embodiment, the processor 104 may be configured to determine whether the PWV value is associated with predefined coefficient values stored in the memory 118 or the external database 126. For example, as discussed previously, the processor 104 may search though the records in the lookup tables 401, 402 using the lookup field. The lookup field, for example, may be the PWV value from the calculation unit 105. The processor 104 may determine whether the PWV value is available in the lookup table 401 or 402, in the affirmative, then using the PWV value as the lookup field, retrieve the regression coefficients 304 in the lookup table 401 or 402 already predefined and associated with the PWV value. For example, the calculation unit 105 executes the calculation program to calculate the PTT value, the arm length and the PWV value based on PTT value and the armlength. Upon calculating, the PWV value, for example, may be "450". The processor 104 may use the PWV value "450" as a lookup field to search and fetch the information from the lookup tables 401, 402 in the external database 126. The processor 104 may retrieve the regression coefficients 304 using the PWV value "450" if there is an entry in the lookup table. The PWV value "450" has an entry in the look-table and the corresponding regression coefficients 304 a=0.023, b=103.57, c=0.025, d=60.77 are retrieved using the PWV value "450" as the lookup field. In this regard, the processor 104 may automatically decide to bypass the calibration unit 112 based on the PWV value since the regression coefficients 304 for the corresponding PWV value is available in the external database 126.

According to an embodiment, the processor 104 may measure the blood pressure of the subject 101 based on the PWV value and predefined coefficient values without performing the calibration. For example, the measurement unit 114 may receive the PWV value from the calculation unit 105 and the regression coefficients 304 from the external database 126 to execute the measurement program using the logic predefined as BP = a^{∗}PWV + b or BP = c^{∗}PWV + d, wherein a, b, c, d are the regression coefficients 304 already predefined in the external database 126 and associated with the PWV value. Regression coefficients a, b may be used for measuring the systolic blood pressure and the regression coefficients c, d may be used for measuring the diastolic blood pressure. In this regard, re-calibrating the subject 101 under varying conditions is obviated since the PWV value of the subject 101 and the associated regression coefficients are readily available to perform the blood pressure measurement.

According to another embodiment, the processor 104 may formulate a decision whether to perform the blood pressure measurement with or without performing the calibration. For example, when the processor 104 may fail to retrieve the regression coefficients 304 using the PWV value from the lookup table, then the processor 104 may automatically decide to perform the calibration. In other words, the processor 104 may instruct the calculation unit 105 to transmit the PWV value to the calibration unit 112 to determine the blood pressure by performing the linear regression 302 on the PWV value and the predefined cuff-based BP values in the memory 118. In such a scenario, the processor 104 may execute all the program logic for calculation, calibration, and measurement in a sequence to determine the blood pressure of the subject 101. In another example, when the processor 104 is able to retrieve the regression coefficients 304 using the PWV value as the lookup field from the lookup table 401 or 402, then the processor 104 may automatically decide to perform the measurement without calibration. In other words, the processor 104 may instruct the calculation unit 105 to transmit the PWV value to the measurement unit 114 to determine the blood pressure using the logic predefined as BP = a^{∗}PWV + b or BP = c^{∗}PWV + d. In such a scenario, the processor 104 may selectively execute the program logic for calculation and measurement in a sequence to determine the blood pressure of the subject 101.

According to an embodiment, the processor 104 may be communicably coupled to the audio interface 121 and the visual interface 120 to receive real-time inputs which can be used in interactive communications with the subject 101. For example, when the processor 104 is unable to identify the PWV value in the lookup table, an interactive communication with the subject 101 may be triggered. The processor 104 may transmit a voice prompt to the audio interface 121 informing the subject 101 or the attendant of the subject 101 that the calibration is required. The subject 101 may respond using verbal commands such as "Yes" or "No". The verbal commands may be processed by a speech recognition engine or a speech recognition application software which converts acoustical signals resulting from spoken words to digital signals recognizable by the processor 104 to trigger the execution of the linear regression model 302 to generate the plurality of coefficients 304. In some examples, the processor 104 may transmit a pop-up screen/window to the visual interface 120 requesting the subject 101 or the attendant of the subject 101 to respond through the touch screen display to trigger the execution of the linear regression model 302. In some examples, the visual interface 120 may provide a blinking screen and the audio interface 121 may provide a beep sound indicative of the calibration or the blood pressure measurement being completed.

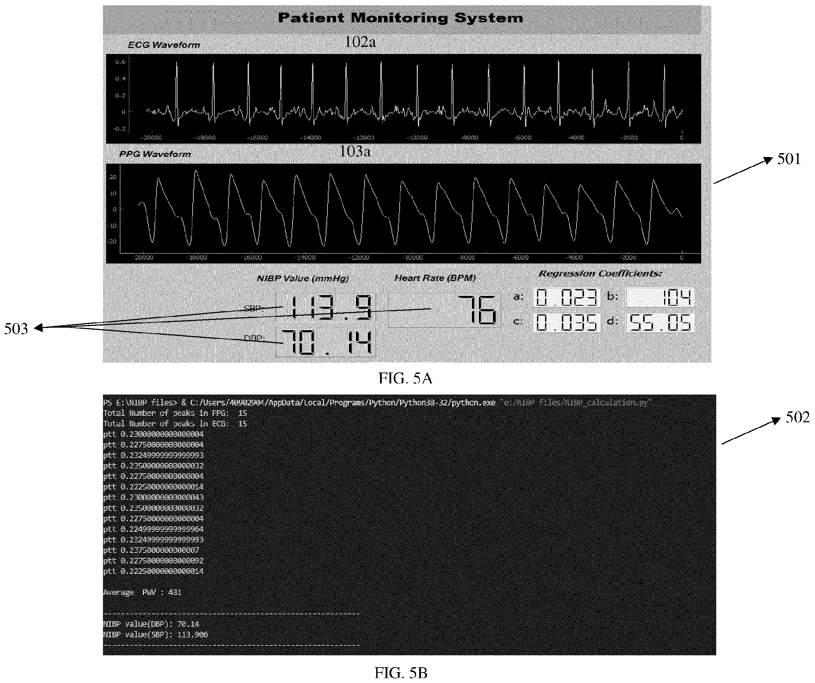

According to another embodiment, the processor 104 may predict the blood pressure of the subject 101 by executing a machine learning or deep learning model. For example, when the processor 104 may fail to retrieve the regression coefficients 304 using the PWV value from the lookup table, then the processor 104 may automatically decide to perform the blood pressure measure using the machine learning or deep learning model. For example, the deep learning model may be based on artificial neural network (ANN) architecture for simultaneously estimating the systolic blood pressure and the diastolic blood pressure. In some examples, the machine or deep learning model may be trained based on multiple pulse flow velocities, one or more predefined blood pressure values, and a plurality of regression coefficients 304. In some examples, the PPG waveform and the ECG waveform may be used to train the model to capture spatial features of the waveforms. In some examples, the training set for training the model may be the records of the lookup table after calibration as shown in FIGS. 4A and 4B. For example, the PWV values, the age, the gender, and the cuff-based BP values may be used to train the machine learning model such that the model may predict the systolic blood pressure and the diastolic blood pressure of the subject 101 when tested with a new PWV value. In another example, the PTT values, the age, the arm length, the gender, and the cuff-based BP values may be used to train the machine learning model such that the model may predict the systolic blood pressure and the diastolic blood pressure of the subject 101 when tested with a new PTT value. It is understood that any type of training dataset may be used to train the machine learning model to predict the blood pressure. Further, the training dataset may include records of multiple subjects under varying conditions to build an accurate model. In this regard, the processor 104 may automatically decide to perform the blood pressure measurement using the machine learning model without calibration or linear regression. FIGS. 5A and 5B disclose examples of user interface screens 501, 502 displayed in the visual interface 120, in accordance with an embodiment of the present disclosure. As shown in FIG. 5A, the user interface screen 501 may display the output of the blood pressure measurement. The output includes live monitoring of the ECG waveform displayed as the first input 102a and the PPG waveform displayed as the second input 103a and live indication of the systolic blood pressure, the diastolic blood pressure, the heart rate, and the regression coefficients. As shown in FIG. 5B, the user interface screen 502 may display additional details such as the total number of peak points in the ECG waveform and the total number of peak points in the PPG waveform. Further, the user interface screen 502 may display the average PWV along with the systolic blood pressure and the diastolic blood pressure. As shown in FIG. 5A, visual indicators 503 representing the values of the systolic blood pressure and the diastolic blood pressure may change color based on the displayed values. For example, the color of the visual indicators 503 may change as per the threshold values of the systolic blood pressure and the diastolic blood pressure provided in the below table:

| **Subject Condition** | **SBP** | **DBP** | **Indicator Colour** |
|---|---|---|---|
| Normal | 90-129 | 60-79 | Green |
| Stage 1 | 130-139 | 80-89 | Yellow |
| Stage 2 | 140-179 | 90-109 | Yellow |
| Critical | Over 180 | Over 110 | Red |

Though FIGS. 5A and 5B depict user interface screens 501, 502 with multiple indicators 503, it is well understood to a person skilled in the art, that the number of indicators 503 may change based on a size of the apparatus in which the blood pressure measurement is implemented. For example, if the apparatus is a smart watch, then only live indication of the systolic blood pressure, the diastolic blood pressure and the heart rate may be displayed in the user interface. For example, if the apparatus is a smart phone, live indication of the systolic blood pressure, the diastolic blood pressure, the heart rate, regression coefficients may be displayed. For example, if the apparatus is any medical device with a larger display screen, then all the indications as shown in FIGS. 5A and 5B may be displayed.

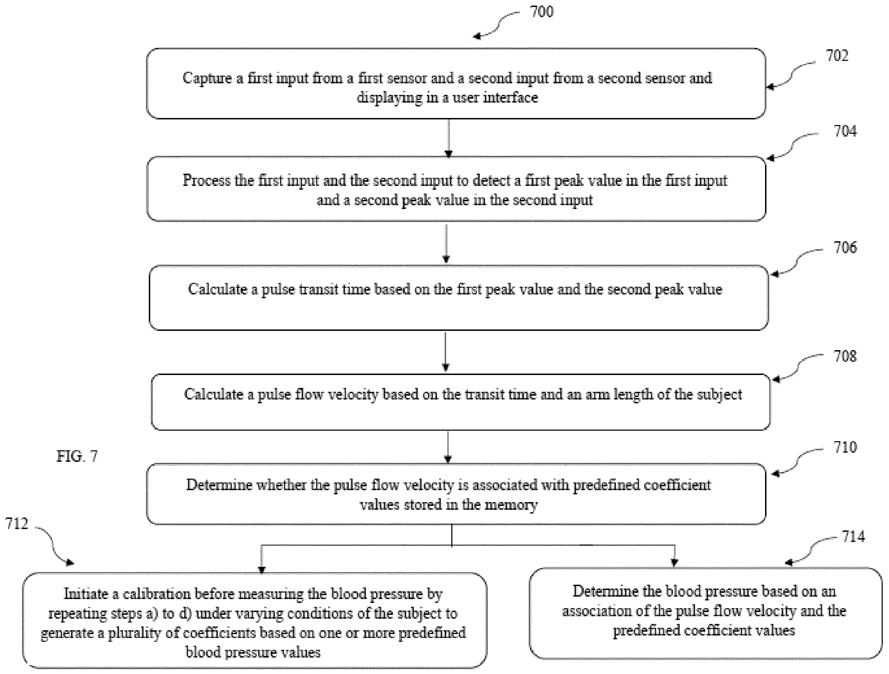

FIG. 7 illustrates a flowchart of discloses a flowchart of a method for non-invasive blood pressure measurement, in accordance with an embodiment of the present disclosure. As illustrated in FIG. 7, the method 700 includes one or more blocks illustrating a method for controlling oxygen supply equipment. The method 700 may be described in the general context of computer executable instructions. Generally, computer executable instructions may include routines, programs, objects, components, data structures, procedures, modules, and functions, which perform specific functions or implement specific abstract data types.

The order in which the method 700 is described is not intended to be construed as a limitation, and any number of the described method blocks may be combined in any order to implement the method. Additionally, individual blocks may be deleted from the methods without departing from the spirit and scope of the subject matter described.

According to an embodiment, the non-invasive blood pressure measurement is executed by a cuffless apparatus. The cuffless apparatus obviated a requirement of using a cuff to measure blood pressure of a subject. The cuffless apparatus may be a standalone apparatus or implemented as a part of any wearable or non-wearable electronic device or another medical device. According to an embodiment, the apparatus includes one or more sensors and a computing system with user interfaces configured to execute the blood pressure measurement. The one or more sensors may be integrated with the computing system. In some examples, the computing system may be a microcontroller unit (MCU) or microprocessor. According to an embodiment, the computing system may include a processor and a memory. The processor may be configured to execute a calculation logic, a calibration logic, and a measurement logic. The steps executed by the processor for calculation, calibration, and measurement in conjunction with the memory, one or more sensors and the user interfaces is disclosed in the FIG. 7.

At step 702, the processor may be configured to capture a first input and a second input from a first sensor and a second sensor respectively. The first sensor and the second sensor are connected to the subject. The first input may be an electrocardiography (ECG) waveform and the second input may be a photoplethysmography (PPG) waveform. In some examples, the first sensor may be an ECG sensor and the second sensor may be a PPG sensor.

At step 704, the processor may be configured to process the first input and the second input to detect a first peak value in the first input and a second peak value in the second input. For example, the first input is displayed as the ECG waveform. The ECG waveform, for example, is a combination of the Q wave, R wave and S wave, commonly known as the "QRS complex". The duration or time interval of the QRS complex may decide conditions of the subject. For example, the second input is displayed as the PPG waveform The PPG waveform, for example, is a combination of peaks and valleys. The first peak value, for example, may be a peak point of the R wave of the QRS complex. The second peak value, for example, may be a systolic peak point of the PPG waveform. In some examples, a synchronization may be performed on peak points for the ECG waveform and the PPG waveform to sequence by occurrence time.

According to an embodiment, the processor may be configured to filter the first input and the second input to detect the first peak value and the second peak value. The filter, for example, may be a band pass filter or a combination of high pass filter and low pass filter. In some examples, the high pass filter may be used for removing a baseline wander which is a low-frequency noise existing in signals (i.e., ECG or PPG) from the first sensor and the second sensor. In some examples, the low pass filter may be used for removing the high-frequency noise existing in signals (i.e., ECG or PPG). Such filtering may be required to remove noise and accurately determine the peak values on the waveforms and ensure that the signals are generated from a same heartbeat cycle or cardiac cycle. At step 706, the processor may be configured to calculate a pulse transit time (PTT) based on the first peak value and the second peak value. The pulse transit time is indicative of a time interval of a pulse pressure wave to travel from one blood vessel to another blood vessel of the subject. The pulse transit time is the difference in time interval between the first peak value and the second peak value obtained from the waveforms. For example, the processor may execute the PTT calculation program in response to detecting the first peak value and the second peak value. For example, the calculation program may include a logic predefined as PTT = diff = Tim (PPG) - Tim (ECG), wherein Tim (ECG) may be the first peak value and the Tim (PPG) may be the second peak value. In other words, the PTT is the time interval between adjacent peak points of the ECG waveform and PPG waveform in the same cardiac cycle

At step 708, the processor may be configured to calculate a pulse wave velocity (PWV) based on the pulse transit time and an arm length of the subject. The pulse wave velocity is indicative of a rate at which the pulse pressure wave travels through the blood vessel. The blood vessel, for example, may be the arteries of the subject. For example, the processor may execute the PWV calculation program in response to detecting the PTT value. For example, the calculation program may include a logic predefined as PWV = Arm Length / PTT, wherein the arm length of the subject is measured using the formula Arm length = 0.55^{∗}height predefined in the calculation program. In some examples, the height value may be received via a voice prompt of the user interface (i.e., an audio interface) requesting the subject or the attendant of the subject to input the height of the subject through verbal utterances. In some examples, the height value may be received via a pop-up screen/window displayed on the user interface (i.e., a visual interface) requesting the subject or the attendant of the subject to input the height of the subject through the touch screen display.

At step 710, the processor may be configured to determine whether the pulse wave velocity is associated with predefined coefficient values stored in the memory. In some examples, the predefined coefficient values may be stored in the external database. In some examples, the predefined coefficient values are regression coefficients obtained from executing a linear regression model prior to measuring the blood pressure. In response to the determination at step 710, the processor may measure the blood pressure of the subject based on the PWV value from the subject and the predefined coefficient values without performing a calibration. In this regard, at step 714, the processor may be configured to determine the blood pressure based on an association of the pulse wave velocity and the predefined coefficient values. The association being predefined in the memory or the external database in the form of look-up tables. For example, the processor may calculate the PWV value from the PTT value and the arm length. Further, the processor may perform search in the lookup tables in the external database or the memory using the PWV value as the lookup field for retrieving records of the regression coefficients from the external database to execute the measurement program for the measuring the blood pressure using the logic predefined as BP = a^{∗}PWV + b or BP = c^{∗}PWV + d, wherein a, b, c, d are the regression coefficients already predefined in the external database and associated with the PWV value. Regression coefficients a, b may be used for measuring the systolic blood pressure and the Regression coefficients c, d may be used for measuring the diastolic blood pressure.

In response to the determination at step 710, when the association between the PWV value and the regression coefficients does not exist, the processor, at step 712, may be configured to initiate a calibration before measuring the blood pressure by repeating steps a) to d) under varying conditions of the subject to generate a plurality of coefficients based on one or more predefined blood pressure values. The steps a) to d) corresponds to steps 702 to 708 which may be repeated under varying conditions of the subject. For example, the conditions of the subject may indicate a different physical activity performed by the subject during the calibration. The physical activity may include, for example, sitting, jogging, walking on a treadmill, riding a bicycle and so on. In this regard, the first input and the second input from the first sensor and the second sensor may vary based on the physical activity performed by the subject. This may result in change in the PTT value and the PWV value. Therefore, the calibration may include recording plurality of PTT values and PWV values under varying conditions of the subject. In this regard, the first input and the second input from the first sensor and the second sensor may vary based on the physical activity performed by the subject. This may result in change in the PTT value and the PWV value. Therefore, the calibration may include recording plurality of PTT values and PWV values under varying conditions of the subject.

According to an embodiment, the processor may perform a linear regression as a part of the calibration on the pulse wave velocity and the one or more predefined blood pressure values to generate the plurality of regression coefficients. In some examples, average PWV may be calculated for calibration when the subject in under varying conditions. For example, about 20 values of PWV may be calculated for the calibration under varying conditions. An average of the 20 values may be determined using PWV=sum(PWV)/len(PWV), wherein the sum(PWV) is a summation of PWV values under varying conditions. Further, a cuff-based equipment can be used to determine the one or more predefined blood pressure values, which can be used by the processor when performing the linear regression to generate the regression coefficients. In this regard, the processor may perform the linear regression on the cuff-based blood pressure values and the average PWV to generate the regression coefficients. In some examples, the processor may store the regression coefficients in the memory in the form of a look up table after performing the linear regression along with an associated to the PWV values. According to an embodiment, the processor may determine the blood pressure after performing the calibration using the logic predefined as BP = a^{∗}PWV + b or BP = c^{∗}PWV + d, wherein a, b, c, d are the regression coefficients already predefined in the external database and associated with the PWV value. Regression coefficients a, b may be used for measuring the systolic blood pressure and the Regression coefficients c, d may be used for measuring the diastolic blood pressure.

According to an embodiment, the processor may display the blood pressure of the subject on the user interface. According to an embodiment, the processor may operate in conjunction with a speech recognition software to issue verbal utterances indicative of the blood pressure of the subject through the user interface. In some examples, the user interface may be an alarm system that may be used to audibly monitor a condition of the human users during an alarm condition such as a sudden rise or fall in the blood pressure measurement. According to an embodiment, the processor may be communicably coupled to the user interface to receive real-time inputs which can be used in execution of the calculation programs or applications running in the computing system. According to an embodiment, the user interface may indicate to the subject if the apparatus or the computing system is performing the calibration and measuring the blood pressure. In some examples, the user interface may provide voice prompts or visual prompts to provide a confirmation on initiating the calibration or the blood pressure measurement or both. In this regard, the computing system or apparatus may be used for both calibration and blood pressure measurement enabling the apparatus to be used on new subjects and re-used on the same subjects.

As will be appreciated by one skilled in the art, aspects of the present invention may be embodied as a system, method, or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer readable program code embodied thereon.

It is to be understood that the software for the computing systems of the present invention embodiments may be implemented in any desired computer language and could be developed by one of ordinary skill in the computer arts based on the functional descriptions contained in the specification and flow charts illustrated in the drawings. By way of example only, the software may be implemented in the C#, C++, Python, Java, or PHP programming languages. Further, any references herein of software performing various functions generally refer to computer systems or processors performing those functions under software control.

Some embodiments may be described using the expression "coupled" and "connected" along with their derivatives. It should be understood that these terms are not intended as synonyms for each other. For example, some embodiments may be described using the term "connected" to indicate that two or more elements are in direct physical or electrical contact with each other. In another example, some embodiments may be described using the term "coupled" to indicate that two or more elements are in direct physical or electrical contact. The term "coupled," however, may also mean that two or more elements are not in direct contact with each other, but yet still cooperate or interact with each other. The embodiments are not limited in this context.

The various embodiments described above are provided by way of illustration only and should not be construed to limit the claims attached hereto. Those skilled in the art will readily recognize various modifications and changes that may be made without following the example embodiments and applications illustrated and described herein, and without departing from the true spirit and scope of the following claims.

## Claims

1. A non-invasive method for measuring a blood pressure of a subject (101) comprising:
a) capturing, by a processor (104), a first input (102a) from a first sensor (102) and a second input (103a) from a second sensor (103), wherein the first sensor (102) and the second sensor (103) are connected to the subject (101);
b) processing, by the processor (104), the first input (102a) and the second input (103a) to detect a first peak value (Tim (ECG)) in the first input (102a) and a second peak value (Tim (PPG)) in the second input (103a);
c) calculating, by the processor (104), a pulse transit time (PTT) based on the first peak value (Tim (ECG)) and the second peak value (Tim (PPG)), wherein the pulse transit time is indicative of a time interval of a pulse pressure wave to travel from one blood vessel to another blood vessel of the subject (101);
d) calculating, by the processor (104), a pulse wave velocity (PWV) based on the pulse transit time and an arm length of the subject (101), wherein the pulse wave velocity is indicative of a rate at which the pulse pressure wave travels through the blood vessel;
e) determining, by the processor (104), whether the pulse wave velocity is associated with predefined coefficient values stored in the memory (118), wherein in response to the determination:
f) initiating, by the processor (104), a calibration before measuring the blood pressure by repeating steps a) to d) under varying conditions of the subject (101) to generate a plurality of coefficients (304) based on one or more predefined blood pressure values; or
g) determining, by the processor (104), the blood pressure based on an association of the pulse wave velocity and the predefined coefficient values.

2. The non-invasive method of claim 1, wherein generating the plurality of coefficients (304) further comprises:
performing, by the processor (104), a linear regression on the pulse wave velocity and the one or more predefined blood pressure values.

3. The non-invasive method of claim 1, further comprises:
filtering, by the processor (104), the first input (102a) and the second input (103a) with a low pass filter and a high pass filter to detect the first peak value (Tim (ECG)) and the second peak value (Tim (PPG)).

4. The non-invasive method of claim 1, further comprises:
displaying, by the processor (104), the blood pressure of the subject (101) on a user interface (120, 121, 501).

5. The non-invasive method of claim 1, wherein the blood pressure is indicative of systolic blood pressure or diastolic blood pressure, wherein the systolic blood pressure (SBP) is calculated by SBP = a^{∗}pulse wave velocity + b, wherein the diastolic blood pressure (DBP) is calculated by DBP = c^{∗}pulse wave velocity + d, and wherein a, b, c, and d are the coefficients (304).

6. An apparatus for non-invasively measuring a blood pressure of a subject (101), the apparatus comprises:
one or more sensors connected to the subject (101) to capture a photoplethysmography waveform and an electrocardiography waveform; and
a processor (104) communicably coupled to the one or more sensors (102, 103) and a memory (118), wherein the processor (104) configured to:
a) process the photoplethysmography waveform and the electrocardiography waveform to detect a first peak value (Tim (ECG)) in the electrocardiography waveform and a second peak value (Tim (PPG)) in the photoplethysmography waveform;
b) calculate a difference in time interval between the first peak value (Tim (ECG)) and the second peak value (Tim (PPG)), wherein the difference in time interval is indicative of a time a pulse pressure wave takes to travel in arteries of the subject (101);
c) calculate a pulse wave velocity (PWV) based on the difference and an arm length of the subject (101), wherein the pulse wave velocity is indicative of a rate at which the pulse pressure wave travels through the arteries; and
d) determine whether the pulse wave velocity is associated with predefined coefficient values stored in the memory (118),
wherein when the association exist,
determine a blood pressure of the subject (101) based on the association of the pulse wave velocity and the predefined coefficient values, and
wherein when the association does not exist,
initiate a calibration by repeating steps a) to c) under varying conditions of the subject (101) to generate a plurality of coefficients (304) based on one or more predefined blood pressure values; and
determine the blood pressure based on a new pulse wave velocity of the subject (101) and the plurality of coefficients (304).

7. The apparatus of claim 6, further comprises: a visual interface (120) to display the blood pressure, wherein the blood pressure is indicative of systolic blood pressure or diastolic blood pressure, and an audio interface (121) to indicate one of the blood pressure is determined and the calibration is initiated or completed.

8. The apparatus of claim 6, wherein the processor (104) is further configured to: predict the blood pressure of the subject (101) by executing a machine learning model trained based on multiple pulse flow velocities, one or more predefined blood pressure values, and a plurality of coefficients (304) when the association does not exist.

9. The apparatus of claim 6, further comprises:
a low pass filter and a high pass filter to eliminate a noise in the photoplethysmography waveform and the electrocardiography waveform to detect the first peak value (Tim (ECG)) and the second peak value (Tim (PPG)).

10. The apparatus of claim 6, wherein the one or more sensor comprises a first sensor (102) comprising: three leads with a first lead LL as ground connected to above waist, a second lead LA as positive connected to left side of subject (101), a third lead RA as a negative connected to the right side of the subject (101), and a second sensor (103) comprising: a light source with a transmitter and a receiver, and wherein the second sensor (103) is connected to one of a finger or a wrist of the subject (101).
